# FASCICULE DE BREVET EUROPEEN

(11) **EP 1 351 654 B1**
(45) Date de publication et mention de la délivrance du brevet: **13.12.2006**
(21) Numéro de dépôt: 02710978.4
(22) Date de dépôt: 11.01.2002
(51) Int. Cl.: A61K 8/81, A61K 8/89, A61Q 5/12

(54) **COMPOSITION CONTENANT AU MOINS UNE SILICONE ET AU MOINS UN POLYMERE AMPHIPHILE**
ZUSAMMENSETZUNG ENTHALTEND MINDESTENS EIN SILICON UND MINDESTENS EIN AMPHIPHILES POLYMER
COMPOSITION CONTAINING AT LEAST ONE SILICONE AND AT LEAST ONE AMPHIPHILIC POLYMER

(30) Priorité: 11.01.2001 FR 0100331
(43) Date de publication de la demande: 15.10.2003
(73) Titulaire: L'ORÉAL, 75008 Paris (FR)
(72) Inventeur: FACK, Géraldine, F-92300 Levallois-Perret (FR); RESTLE, Serge, F-95390 Saint-Prix (FR)
(74) Mandataire: Le Blainvaux Bellegarde, Françoise
(86) Numéro de dépôt international: PCT/FR2002/000102
(87) Numéro de publication internationale: WO 2002/055032

(56) Documents cités:
- EP-A- 0 406 042
- EP-A- 0 750 899
- EP-A- 0 815 843
- EP-A- 1 055 406
- WO-A-00/31154
- WO-A-98/56333
- US-A- 4 460 732
- US-A- 4 861 499
- US-A- 5 089 578
- US-A- 5 114 706
- US-A- 5 464 452
- A KOBAYASHI ET ALL: "Solubilization Properties of N-substituted Amphiphilic Acrylamide Copolymers" JOURNAL OF APPLIED POLYMER SCIENCE., vol. 73, no. 12, 19 septembre 1999 (1999-09-19), pages 2447-2453, XP002177425 JOHN WILEY AND SONS INC. NEW YORK., US ISSN: 0021-8995
- PATENT ABSTRACTS OF JAPAN vol. 1997, no. 02, 28 février 1997 (1997-02-28) & JP 08 252447 A (SHISEIDO CO LTD), 1 octobre 1996 (1996-10-01)

## Description

La présente invention se rapporte à une composition pour application topique, comprenant au moins une silicone et au moins un polymère amphiphile, et à son utilisation notamment pour le traitement et le soin de la peau humaine, du cuir chevelu, des muqueuses, des ongles et des cheveux.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légéreté du cheveu), un manque de lissage (cheveu non homogène de la racine à la pointe).

Compte tenu du caractère généralement insoluble des silicones utilisables dans les compositions de lavage et de conditionnement, on cherche à maintenir les silicones en dispersion régulière dans le milieu sans cependant faire chuter la viscosité et les propriétés des compositions. Les silicones doivent également être véhiculées sur les matières kératiniques traitées en vue de leur conférer, suivant l'application, des propriétés de douceur, de brillance et de démêlage.
Il existe à ce jour peu de moyens pour maintenir efficacement en suspension les silicones insolubles, car il s'agit là d'un problème difficile à résoudre ; à cet effet, on a déjà proposé l'utilisation des dérivés d'esters ou d'éthers à longue chaîne (agents nacrants) ou des polysaccharides tels que la gomme de xanthane (gélifiants). Cependant, les agents nacrants présentent des problèmes de cristallisation qui entraînent une évolution (augmentation) de la viscosité des compositions au cours du temps ; les agents gélifiant présentent également des inconvénients, à savoir, d'une part que la mousse des compositions détergentes contenant de la gomme de xanthane se développe difficilement (mauvais démarrage de mousse) et que, d'autre part, les compositions n'ont pas une texture lisse et s'écoulent par paquets, ce qui est peu apprécié des utilisateurs.

En outre, ces compositions laissent la peau « comme mouillée » après application, ces compositions ne pénétrant pas suffisamment dans la peau. Tout ceci rend leur utilisation rédhibitoire dans les domaines cosmétique et/ou dermatologique.

Par ailleurs, les copolymères anioniques réticulés comportant des groupements 2-acrylamidô 2-méthyl propane sulfonique, tels que le produit commercialisé par la société SEPPIC sous la dénomination SEPIGEL 305 (constitué de motifs dérivant de la réaction entre (i) l'acrylamide, (ii) l'acide 2-acrylamido 2-méthyl propane sulfonique et (iii) au moins un composé à polyinsaturation oléfinique) ne permettent pas d'obtenir des compositions se répartissant de façon homogène et présentant une bonne rinçabilité et un bon dépôt de la silicone sur les matières kératiniques .

En outre, si l'utilisation des homopolymères d'acide 2-acrylamido 2-méthylpropane sulfonique (AMPS), tels que le produit commercialisé par la société Clariant sous la dénomination Hostacerin AMPS permet d'obtenir la gélification de milieux aqueux tout en gardant de bonnes propriétés cosmétiques, par rapport à d'autres gélifiants disponibles, les compositions se répartissent mal sur l'ensemble de la chevelure et se rincent également difficilement.

Il subsiste donc le besoin de compositions contenant des silicones et ne présentant pas les inconvénients de l'art antérieur.

La demanderesse a trouvé de façon inattendue un polymère amphiphile permettant d'une part de stabiliser les compositions contenant une silicone, notamment sans entraîner la fluidification ou l'épaississement des dites compositions ou le relarguage de ladite silicone et d'autre part d'obtenir des compositions se répartissant facilement sur les cheveux, se rinçant facilement sans laisser un toucher désagréable sur les cheveux. Par ailleurs, la silicone se dépose bien sur le cheveu.

Aussi, la présente invention a pour objet une composition pour application topique, comportant une phase aqueuse comprenant au moins une silicone choisie parmi les résines de silicones non renforcées, les polyorganosiloxanes modifiés par des groupements organofonctionnels non polyoxyalkylénés ainsi que leurs mélanges et au moins un polymère amphiphile comportant au moins un monomère à insaturation éthylénique à groupement sulfonique, sous forme libre ou partiellement ou totalement neutralisée et comprenant au moins une partie hydrophobe.

La composition de l'invention étant destinée à une application topique, elle comprend un milieu physiologiquement acceptable. On entend dans la présente demande par « milieu physiologiquement acceptable » un milieu compatible avec toutes les matières kératiniques telles que la peau y compris le cuir chevelu, les ongles, les muqueuses, les yeux et les cheveux ou toute autre zone cutanée du corps.

La composition obtenue présente une excellente stabilité et conserve une viscosité satisfaisante (sans fluidication ou épaississement incontrôlé ou relargage de la silicone), même en présence d'une quantité importante de silicone.

Aussi, la présente invention a également pour objet l'utilisation d'un polymère amphiphile comportant au moins un monomère à insaturation éthylènique à groupement sulfonique, sous forme libre ou partiellement ou totalement neutralisée et comprenant au moins une partie hydrophobe, pour stabiliser une composition cosmétique ou dermatologique contenant au moins une silicone choisie parmi les résines de silicones non renforcées, les polyorganosiloxanes modifiés par des groupements organofonctionnels non polyoxyalkylénés ainsi que leurs mélanges et/ou augmenter son dépôt.

Un autre objet de l'invention concerne l'utilisation d'un polymère amphiphile comportant au moins un monomère à insaturation éthylènique à groupement sulfonique, sous forme libre ou partiellement ou totalement neutralisée et comprenant au moins une partie hydrophobe, dans, ou pour la fabrication d'une composition cosmétique comprenant une silicone choisie parmi les résines de silicones non renforcées, les polyorganosiloxanes modifiés par des groupements organofonctionnels non polyoxyalkylénés ainsi que leurs mélanges.

Les différents objets de l'invention vont maintenant être détaillés. L'ensemble des significations et définitions des composés utilisés dans la présente invention données ci-dessous sont valables pour l'ensemble des objets de l'invention.

La composition obtenue a la viscosité souhaitée, c'est-à-dire généralement supérieure à 0,5 poise, par exemple de 1 à 50 poises (0,1 à 5 Pa.s), et de préférence de 5 à 25 poises. (0,5 à 2,5 Pa.s), ladite viscosité étant mesurée à la température ambiante (environ 25°C) avec un Rhéomat 180 à l'aide d'un mobile 2, 3, 4 ou 5 selon la gamme de viscosité, à 200 s⁻¹.

La composition de l'invention peut être une dispersion aqueuse, un gel aqueux ou hydroalcoolique, contenant ou non une huile, ainsi qu'une émulsion eau-dans-huile ou huile-dans-eau.

Les polymères conformes à l'invention sont des polymères amphiphiles comportant au moins un monomère à insaturation éthylènique à groupement sulfonique, sous forme libre ou partiellement ou totalement neutralisée et comprenant au moins une partie hydrophobe.
On entend par polymère amphiphile, tout polymère comportant à la fois une partie hydrophile et une partie hydrophobe et notamment une chaîne grasse.

La partie hydrophobe présente dans les polymères de l'invention comporte de préférence de 6 à 50 atomes de carbone, plus préférentiellement de 6 à 22 atomes de carbone, plus préférentiellement encore de 6 à 18 atomes de carbone, et plus particulièrement de 12 à 18 atomes de carbone.

De façon préférentielle, les polymères conformes à l'invention sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Les polymères amphiphiles conformes à l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Les polymères amphiphiles selon l'invention peuvent être réticulés ou non-réticulés.

De préférence, on choisit des polymères amphiphiles réticulés. Lorsqu'ils sont réticulés, les agents de réticulation peuvent être choisis parmi les composés à polyinsaturation oléfinique couramment utilisés pour la réticulation des polymères obtenus par polymérisation radicalaire.
On peut citer par exemple, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

On utilisera plus particulièrement le méthylène-bis-acrylamide, le méthacrylate d'allyle ou le triméthylol propane triacrylate (TMPTA). Le taux de réticulation variera en général de 0,01 à 10% en mole et plus particulièrement de 0,2 à 2% en mole par rapport au polymère.

On utilisera l'acide 2-acrylamido-2-méthylpropane-sulfonique (AMPS) ainsi que ses formes partiellement ou totalement neutralisées, en tant que monomère à insaturation éthylènique.

Les polymères amphiphiles conformes à l'invention peuvent notamment être choisis parmi les polymères amphiphiles statistiques d'AMPS modifiés par réaction avec une n-monoalkylamine ou une di-n-alkylamine en C₆-C₂₂, et tels que ceux décrits dans la demande de brevet WO00/31154 (faisant partie intégrante du contenu de la description). Ces polymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou polyalkylèneglycols, les (méth)acrylamides, la vinylpyrrolidone, l'anhydride maléique, l'acide itaconique ou l'acide maléique ou les mélanges de ces composés.

Les polymères préférés de l'invention sont choisis parmi les copolymères amphiphiles d'AMPS et d'au moins un monomère hydrophobe à insaturation éthylénique comportant au moins une partie hydrophobe ayant de 6 à 50 atomes de carbone et plus préférentiellement de 6 à 22 atomes de carbone et encore plus préférentiellement de 6 à 18 atomes de carbone et plus particulièrement 12 à 18 atomes de carbone.
Ces mêmes copolymères peuvent contenir en outre un ou plusieurs monomères éthyléniquement insaturés ne comportant pas de chaîne grasse tels que les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans la demande de brevet EP-A-750899, le brevet US 5089578 et dans les publications de Yotaro Morishima suivantes :
- « Self-assembling amphiphilic polyelectrolytes and their nanostructures - Chinese Journal of Polymer Science Vol. 18, N°40, (2000), 323-336. »
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
- « Solution properties of miscelle networks formed by non-ionic moieties covalently bound to an polyelectrolyte : salt effects on rheological behavior - Langmuir, 2000, Vol.16, N°12, 5324-5332 » ;
- « Stimuli responsive amphiphilic copolymers of sodium 2-(acrylamido)-2-methylpropanesulfonate and associative macromonomers - Polym. Preprint, Div: Polym. Chem. 1999, 40(2), 220-221 ».

Les monomères hydrophobes à insaturation éthylénique des copolymères sont choisis parmi les acrylates ou les acrylamides de formule (I) suivante : 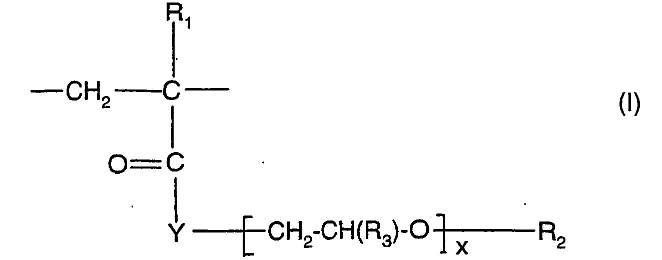 dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné hydrophobe comportant au moins de 6 à 50 atomes de carbone et plus préférentiellement de 6 à 22 atomes de carbone et encore plus préférentiellement de 6 à 18 atomes de carbone et plus particulièrement de 12 à 18 atomes de carbone ; x désigne un nombre de moles d'oxyde d'alkylène et varie de 3 à 100.

Le radical R₂ est choisi de préférence parmi les radicaux alkyles en C₆-C₁₈ linéaires (par exemple n-hexyle, n-octyle, n-décyle, n-hexadécyle, n-dodécyle), ramifiés ou cycliques (par exemple cyclododécane (C₁₂) ou adamantane (C₁₀)) ; les radicaux alkylperfluorés en C₆-C₁₈ (par exemple le groupement de formule - (CH₂)₂-(CF₂)₉-CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Parmi ces polymères, on peut citer :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(méth)acrylate par rapport au polymère, tels que ceux décrits dans la demande EP-A750 899 ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, tels que ceux décrits dans le brevet US-5089578.

On citera plus particulièrement les copolymères constitués de motifs acide 2-acrylamido-2-méthylpropane-sulfonique (AMPS) de formule (II) suivante : 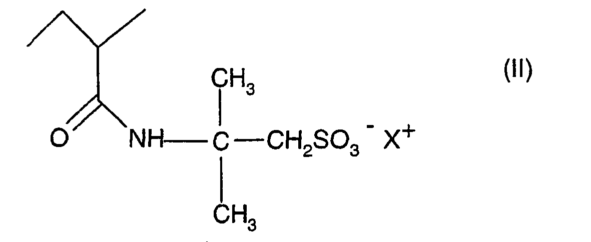 dans laquelle X⁺ est un proton, un cation de métal alcalin, un cation alcalino-terreux ou l'ion ammonium,
et de motifs de formule (III) suivante : 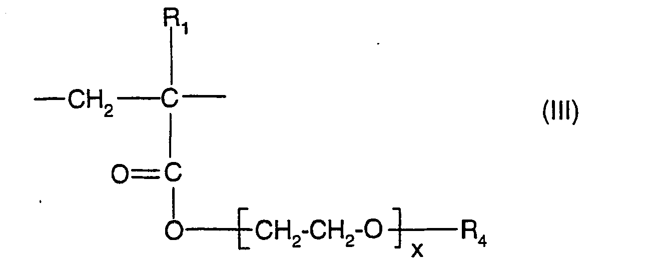 dans laquelle x désigne un nombre entier variant de 3 à 100, de préférence de 5 à 80 et plus préférentiellement de 7 à 25 ; R₁ a la même signification que celle indiquée ci-dessus dans la formule (I) et R₄ désigne un alkyle linéaire ou ramifié en C₆-C₂₂ et plus préférentiellement en C₁₀-C₂₂.

Les polymères particulièrement préférés sont ceux pour lesquels x = 25 , R₁ désigne méthyle et R₄ représente n-dodécyle ; ils sont décrits dans les articles de

Morishima mentionnés ci-dessus.
Les polymères pour lesquels X⁺ désigne sodium ou ammonium sont plus particulièrement préférés.

Les polymères amphiphiles préférés conformes à l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le ABAH (2,2-azobis-[2-amidinopropane] hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ils sont notamment obtenus par polymérisation radicalaire en milieu tert-butanol dans lequel ils précipitent.
En utilisant la polymérisation par précipitation dans le tert-butanol, il est possible d'obtenir une distribution de la taille des particules du polymère particulièrement favorable pour ses utilisations.
La distribution de la taille des particules du polymère peut être déterminée par exemple par diffraction laser ou analyse d'image.
Une distribution intéressante pour de type de polymère et déterminée par analyse d'image est la suivante: 60,2% inférieur à 423 microns, 52,0% inférieur à 212 microns, 26,6% inférieur à 106 microns, 2,6% inférieur à 45 microns et 26,6% supérieur à 850 microns.
La réaction peut être conduite à une température comprise entre 0 et 150°C, de préférence entre 10 et 100°C , soit à pression atmosphérique, soit sous pression réduite. Elle peut aussi être réalisée sous atmosphère inerte, et de préférence sous azote.
Selon ce procédé, on a notamment polymérisé l'acide 2-acrylamido-2-méthylpropane-sulfonique (AMPS) ou l'un de ses sels de sodium ou d'ammonium, avec un ester de l'acide (méth)acrylique et,
- d'un alcool en C₁₀-C₁₈ oxyéthyléné par 8 moles d'oxyde d'éthylène (GENAPOL^{®} C-080 de la société HOECHST/CLARIANT),
- d'un alcool oxo en C₁₁ oxyéthyléné par 8 moles d'oxyde d'éthylène (GENAPOL^{®} UD-080 de la société HOECHST/CLARIANT),
- d'un alcool oxo en C₁₁ oxyéthyléné par 7 moles d'oxyde d'éthylène (GENAPOL^{®} UD-070 de la société HOECHST/CLARIANT),
- d'un alcool en C₁₂-C₁₄ oxyéthyléné par 7 moles d'oxyde d'éthylène (GENAPOL^{®} LA-070 de la société HOECHST/CLARIANT),
- d'un alcool en C₁₂-C₁₄ oxyéthyléné par 9 moles d'oxyde d'éthylène (GENAPOL^{®} LA-090 de la société HOECHST/CLARIANT),
- d'un alcool en C₁₂-C₁₄ oxyéthyléné par 11 moles d'oxyde d'éthylène (GENAPOL^{®} LA-110 de la société HOECHST/CLARIANT),
- d'un alcool en C₁₆-C₁₈ oxyéthyléné par 8 moles d'oxyde d'éthylène (GENAPOL^{®} T-080 de la société HOECHST/CLARIANT),
- d'un alcool en C₁₆-C₁₈ oxyéthyléné par 15 moles d'oxyde d'éthylène (GENAPOL^{®} T-150 de la société HOECHST/CLARIANT),
- d'un alcool en C₁₆-C₁₈ oxyéthyléné par 11 moles d'oxyde d'éthylène (GENAPOL^{®} T-110 de la société HOECHST/CLARIANT),
- d'un alcool en C₁₆-C₁₈ oxyéthyléné par 20 moles d'oxyde d'éthylène (GENAPOL^{®} T-200 de la société HOECHST/CLARIANT),
- d'un alcool en C₁₆-C₁₈ oxyéthyléné par 25 moles d'oxyde d'éthylène (GENAPOL^{®} T-250 de la société HOECHST/CLARIANT),
- d'un alcool en C₁₈-C₂₂ oxyéthyléné par 25 moles d'oxyde d'éthylène et/ou d'un alcool iso C₁₆-C₁₈ oxyéthyléné par 25 moles d'oxyde d'éthylène.

La concentration molaire en % des motifs de formule (II) et des motifs de formule (III) dans les polymères selon l'invention variera en fonction de l'application cosmétique souhaitée et des propriétés rhéologiques de la formulation recherchées. Elle peut varier entre 0,1 et 99,9% en moles.
De préférence pour les polymères les plus hydrophobes, la proportion molaire en motifs de formule (I) ou (III) varie de 50,1 à 99,9%, plus particulièrement de 70 à 95% et encore plus particulièrement de 80 à 90%.
De préférence pour les polymères peu hydrophobes, la proportion molaire en motifs de formule (I) ou (III) varie de 0,1 à 50%, plus particulièrement de 5 à 25% et encore plus particulièrement de 10 à 20%.
La distribution des monomères dans les polymères de l'invention peut être, par exemple, alternée, bloc (y compris multibloc) ou quelconque.
Selon l'invention, on préfère que les polymères aient des chaînes pendantes sensibles à la chaleur et dont la solution aqueuse présente une viscosité qui, au delà d'une certaine température seuil, augmente, ou demeure pratiquement constante quand la température augmente.
Plus particulièrement encore, on préfère les polymères dont la solution aqueuse présente une viscosité qui est faible en dessous d'une première température seuil et qui, au dessus de cette première température seuil croît vers un maximum quand la température augmente, et qui, au dessus d'une seconde température seuil décroît à nouveau quand la température augmente. Dans cet optique, on préfère que la viscosité des solutions de polymère en dessous de la première température seuil soit de 5 à 50%, en particulier de 10 à 30% de la viscosité maximum à la seconde température seuil.
Ces polymères, de préférence, conduisent dans l'eau à un phénomène de démixion par chauffage se traduisant par des courbes présentant, en fonction de la température et de la concentration, un minimum appelé LCST (Lower Critical

Solution Temperature).

Les viscosités (mesurées à 25°C au viscosimètre Brookfield aiguille 7) des solutions aqueuses à 1 % vont de préférence de 20 000 mPa.s à 100 000 mPa.s et plus particulièrement de 60 000 mPa.s à 70 000 mPa.s.

Les polymères amphiphiles conformes à l'invention sont présents dans les compositions dans des concentrations allant de 0,01 à 30% en poids, plus préférentiellement de 0,1 à 10%, encore plus préférentiellement de 0,1 à 5% en poids et plus particulièrement encore de 0,5 à 2% en poids.

Les silicones peuvent être présentes dans la composition de l'invention en une quantité allant par exemple de 0,01 à 20 % en poids, et de préférence de 0,01 à 10 % et mieux de 0,05 à 5 % en poids par rapport au poids total de la composition.

Les silicones utilisables conformément à l'invention peuvent être solubles ou insolubles dans l'eau ou la composition.

Les silicones utilisables conformément à l'invention sont en particulier des polyorganosiloxanes insolubles dans la composition et peuvent se présenter sous forme d'huiles, de cires, de résines ou de gommes.

Les silicones insolubles dans l'eau sont insolubles dans l'eau à une concentration supérieure ou égale à 0,1% en poids dans l'eau à 25°C, c'est à dire qu'elles ne forment pas une solution isotrope transparente.

La viscosité des silicones est par exemple mesurée à 25°C selon la norme ASTM 445 Appendice C.

Les organopolysiloxanes sont définis plus en détail dans l'ouvrage de Walter NOLL "Chemistry and Technology of Silicones" (1968) Academie Press. Elles peuvent être volatiles ou non volatiles.

On utilise de préférence des silicones non volatiles et plus particulièrement des résines de silicones non renforcées (en particulier par de la silice), des polyorganosiloxanes modifiés par des groupements organofonctionnels non polyoxyalkylénés ainsi que leurs mélanges.

Parmi ces polyalkylsiloxanes, on peut citer à titre non limitatif les produits commerciaux commercialisés sous les dénominations "ABIL WAX 9800 et 9801" par la société GOLDSCHMIDT qui sont des polyalkyl (C₁-C₂₀) siloxanes.

Les résines de silicone utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle.
Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées non polyoxyalkylénées utilisables conformément à l'invention sont des polysiloxanes en particulier des polydiméthylsiloxanes comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;

On peut ainsi citer :
(a) les polysiloxanes dénommés dans le dictionnaire CTFA "amodiméthicone" et répondant à la formule : 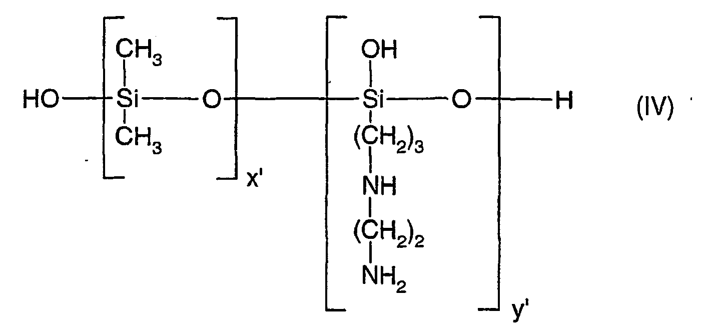 dans laquelle x' et y' sont des nombres entiers dépendant du poids moléculaire, généralement tels que ledit poids moléculaire moyen en poids est compris entre 5 000 et 500 000 environ ;
(b) les silicones aminées répondant à la formule :

   R'ₐG₃₋ₐSi(OSiG₂)ₙ-(OSiG_{b}R'_{2-b})ₘ-O-SiG₃₋ₐ-R'ₐ (V)

   dans laquelle :
   G est un atome d'hydrogène, ou un groupement phényle, OH, ou alkyle en C₁-C₈, par exemple méthyle,
   a désigne le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R' est un radical monovalent de formule -C_{q}H_{2q}L dans laquelle q est un nombre de 2 à 8 et L est un groupement aminé éventuellement quaternisé choisi parmi les groupements :

      -NR"-CH₂-CH₂-N'(R")₂

      -N(R")₂

      -N^{⊕}(R")₃ A⁻

      -NH^{⊕}(R")₂ A⁻

      -NH₂^{⊕}(R")A⁻

      -N(R")-CH₂-CH₂-N^{⊕}R" H₂ A⁻,
   dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.
   Un produit correspondant à cette définition est la silicone dénommée "triméthylsilylamodiméthicone", répondant à la formule : 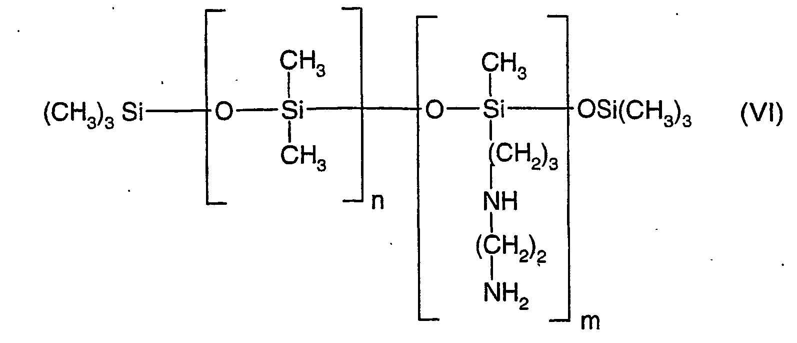 dans laquelle n et m ont les significations données ci-dessus (cf formule V).
   De tels polymères sont décrits par exemple dans la demande de brevet EP-A-95238.
(c) les silicones aminées répondant à la formule : 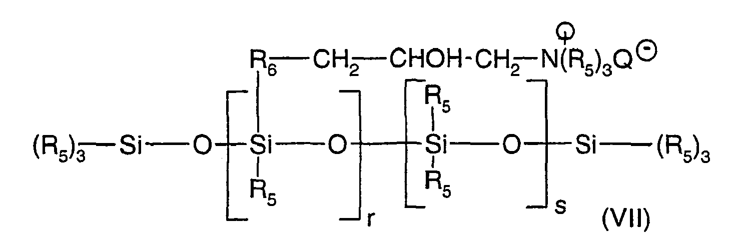 dans laquelle
   R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   Q⁻ est un anion tel qu'un ion halogénure, notamment chlorure ou un sel d'acide organique (acétate...);
   r représente une valeur statistique moyenne de 2 à 20 et en particulier de 2 à 8 ;
   s représente une valeur statistique moyenne de 20 à 200 et en particulier de 20 à 50.
   De tels silicones aminées sont décrites plus particulièrement dans le brevet US 4185 087.
   Une silicone entrant dans cette classe est la silicone commercialisée par la société Union Carbide sous la dénomination "Ucar Silicone ALE 56.
d) les silicones ammonium quaternaire de formule : 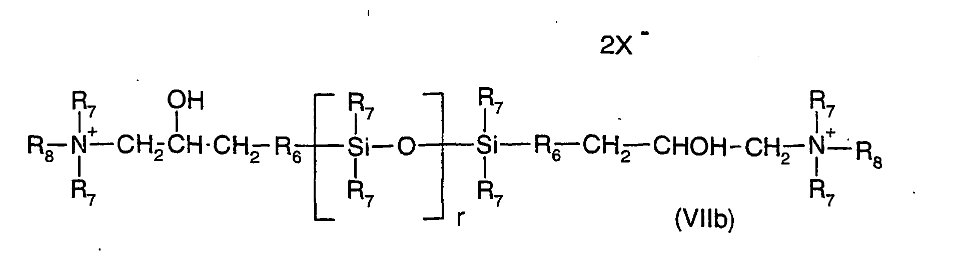 dans laquelle
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
   X- est un anion tel qu'un ion halogénure, notamment chlorure ou un sel d'acide organique (acétate...);
   r représente une valeur statistique moyenne de 2 à 200 et en particulier de 5 à 100 ;

Ces silicones sont par exemple décrites dans la demande EP-A-0530974.

Des silicones entrant dans cette classe sont les silicones commercialisées par la société GOLDSCHMIDT sous les dénominations ABIL QUAT 3270, ABIL QUAT 3272, ABIL QUAT 3474.

Selon l'invention, les silicones aminées peuvent se présenter sous formes d'huile, de solutions aqueuses, alcooliques ou hydroalcooliques, sous forme de dispersion ou d'émulsion.

Une forme de réalisation particulièrement intéressante est leur utilisation sous forme d'émulsions en particulier sous forme de microémulsions ou de nanoémulsions.

On peut utiliser par exemple le produit commercialisé sous la dénomination "Emulsion Cationique DC 929" par la Société Dow Corning qui comprend, outre l'amodiméthicone, un agent de surface cationique dérivés des acides gras du suif dénommé Tallowtrimonium(CTFA), en association avec un agent de surface non ionique connu sous la dénomination "Nonoxynol 10".

On peut également utiliser par exemple le produit commercialisé sous la dénomination "Emulsion Cationique DC 939" par la Société Dow Corning qui comprend, outre l'amodiméthicone, un agent de surface cationique le chlorure de triméthyl cétyl ammonium en association avec un agent de surface non ionique le tridéceth-12.

Un autre produit commercial utilisable selon l'invention est le produit commercialisé sous la dénomination "Dow Corning Q2 7224" par la Société Dow Corning comportant en association le triméthylsilylamodiméthicone de formule (IV), un agent de surface non ionique de formule : C₈H₁₇-C₆H₄-(OCH₂CH₂)ₙ-OH où n = 40 dénommé encore octoxynol-40,
un autre agent de surface non ionique de formule : C₁₂H₂₅-(OCH₂-CH₂)ₙ-OH où n = 6 encore dénommé isolaureth-6, et du glycol.
- des groupements thiols comme les produits commercialisés sous les dénominations "GP 72 A" et "GP 71" de GENESEE ;
- des groupements alcoxylés pendants comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés pendants comme les polyorganosiloxanes à fonction hydroxyalkyle décrits dans la demande de brevet français FR-A-85 16334 répondant à la formule (IV) : 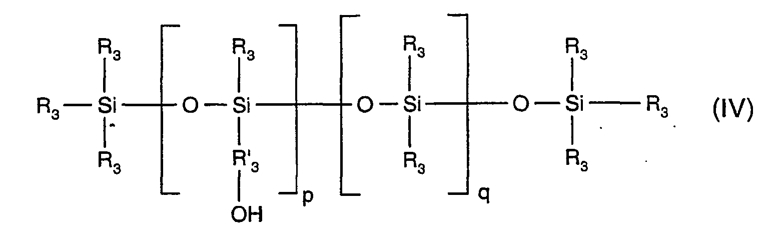 dans laquelle les radicaux R₃ identiques ou différents sont choisis parmi les radicaux méthyle et phényle ; au moins 60 % en mole des radicaux R₃ désignant méthyle ; le radical R'₃ est un chaînon alkylène divalent hydrocarboné en C₂-C₁₈ ; p est compris entre 1 et 30 inclus ; q est compris entre 1 et 150 inclus ;
- des groupements acyloxyalkyle tels que par exemple les polyorganosiloxanes décrits dans le brevet US-A-4957732 et répondant à la formule (V) : 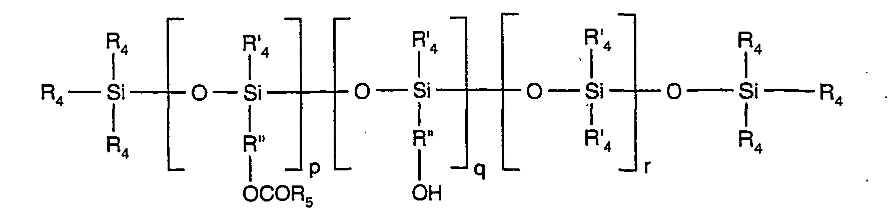 dans laquelle :
   R₄ désigne un groupement méthyle, phényle, -OCOR₅, hydroxyle, un seul des radicaux R₄ par atome de silicium pouvant être OH ;
   R'₄ désigne méthyle, phényle ; au moins 60 % en proportion molaire de l'ensemble des radicaux R₄ et R'₄ désignant méthyle ;
   R₅ désigne alkyle ou alcényle en C₈-C₂₀ ;
   R" désigne un radical alkylène hydrocarboné divalent, linéaire ou ramifié en C₂-C₁₈ ;
   r est compris entre 1 et 120 inclus ;
   p est compris entre 1 et 30 ;
   q est égal à 0 ou est inférieur à 0,5 p, p + q étant compris entre 1 et 30 ; les polyorganosiloxanes de formule (VI) peuvent contenir des groupements : 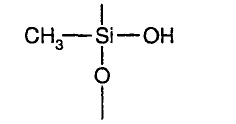 dans des proportions ne dépassant pas 15 % de la somme p + q + r.
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule (VII): 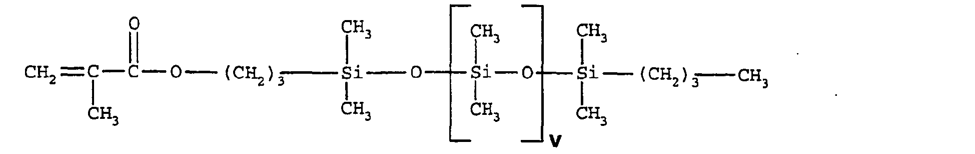 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions.

Les silicones particulièrement préférés conformément à l'invention sont :
- les résines de silicone non renforcées,
- les polysiloxanes à groupements aminés tels que les amodiméthicones ou les triméthylsilylamodiméthicones ;

Le milieu physiologiquement acceptable de la composition de l'invention comprend de l'eau. La quantité d'eau peut aller de 30 à 99,94 %, en poids et de préférence de 30 à 95 % en poids par rapport au poids total de la composition. Ce milieu peut contenir, outre l'eau, un ou plusieurs solvants choisis parmi les alcools inférieurs comportant de 1 à 8 atomes de carbone, tels que l'éthanol ; les polyols tels que la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose, le sucrose ; et leurs mélanges. La quantité de solvant(s) dans la composition de l'invention peut aller de 0,5 à 30 % en poids et de préférence de 5 à 20 % en poids par rapport au poids total de la composition.

La composition de l'invention a de préférence un pH compatible avec les matières kératiniques, c'est-à-dire allant de préférence de 3 à 10 et mieux de 4 à 7.

La composition de l'invention peut contenir également un ou plusieurs des additifs habituels dans le domaine cosmétique et/ou dermatologique, tels que les charges minérales ou organiques, les actifs, les conservateurs, les gélifiants, les plastifiants, les antioxydants, les parfums, les absorbeurs d'odeur, les agents antimousse, les séquestrants (EDTA), les ajusteurs de pH acides ou basiques ou des tampons, les pigments et les nacres, les corps gras comme les huiles ou les cires, et leurs mélanges, dans la mesure où l'additif n'altère pas les propriétés recherchées pour la composition de l'invention. Les quantités de ces différents additifs sont celles classiquement utilisées dans les domaines considérés, et par exemple de 0,01 à 20 % du poids total de la composition.

La composition selon l'invention peut se présenter sous toutes les formes galéniques appropriées pour une application topique, et notamment sous forme de gel aqueux ou hydroalcoolique, d'émulsion eau-dans-huile (E/H) ou huile-dans-eau (H/E) ou multiple (E/H/E ou H/E/H), ou de dispersion aqueuse à base de vésicules lipidiques ioniques et/ou non-ioniques, contenant ou non une huile dispersée. Elle peut constituer par exemple un sérum, une crème ou un lait, une lotion épaissie ou une mousse.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse.

Lorsque la composition de l'invention est une émulsion, la proportion de la phase grasse (ou phase huileuse) peut aller de 5 à 80 % en poids, et de préférence de 10 à 50 % en poids par rapport au poids total de la composition et la proportion de phase aqueuse peut aller de 20 à 95 % et de préférence de 50 à 90 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsion peut éventuellement être préparée sans addition d'émulsionnants, grâce au caractère amphiphile du polymère. Quand l'émulsion contient un émulsionnant, celui-ci et le coémulsionnant éventuellement présent sont présents en une proportion allant de 0,1 à 30 % en poids, et de préférence de 0,3 à 20 % en poids par rapport au poids total de la composition. L'émulsion peut, en outre, contenir des vésicules lipidiques.

Comme huiles utilisables dans l'invention, on peut citer les huiles minérales (isoparaffine hydrogénée), les huiles d'origine végétale (huile d'abricot), les huiles d'origine animale, les huiles de synthèse, et les huiles fluorées.

Les autres corps gras pouvant être présents dans la phase huileuse sont par exemple les acides gras comportant de 8 à 30 atomes de carbone, comme l'acide stéarique, l'acide laurique, l'acide palmitique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch.

La composition selon l'invention peut être utilisée pour le traitement et le soin de la peau du visage et/ou du corps, des muqueuses (lèvres), du cuir chevelu et/ou des cheveux, et aussi pour le traitement de la peau sensible et/ou du cuir chevelu sensible.

Aussi, la présente invention a pour objet l'utilisation cosmétique de la composition cosmétique telle que définie ci-dessus, pour le traitement et le soin de la peau du visage et/ou du corps, des muqueuses, du cuir chevelu et/ou des cheveux.

La présente invention a encore pour objet l'utilisation de la composition telle que définie ci-dessus pour la fabrication d'une composition destinée à traiter la peau sensible et/ou le cuir chevelu sensible. Pour de plus amples détails concernant la peau sensible, on peut se référer au document EP-A-680749.

La composition selon l'invention peut constituer par exemple une crème de soin du visage, un lait corporel, un gel de douche, un gel de bain, une composition de coloration ou de décoloration des cheveux, une composition de déformation permanente des cheveux, une composition de nettoyage et/ou de démaquillage du visage, une composition de protection solaire. Les compositions selon l'invention peuvent être également des compositions de shampooing, d'après-shampooing à rincer ou non.

Les compositions de l'invention peuvent également se présenter sous forme de compositions à rincer, à appliquer avant ou après une coloration, une décoloration, une permanente ou un défrisage ou encore entre les deux étapes d'une permanente ou d'un défrisage.

La présente invention a aussi pour objet un procédé de traitement cosmétique de la peau, du cuir chevelu, des cheveux, et/ou des muqueuses, caractérisé par le fait que l'on applique sur la peau, le cuir chevelu, les cheveux, et/ou les muqueuses, une composition telle que définie ci-dessus.

Les exemples ci-après de compositions selon l'invention, sont donnés à titre d'illustration et sans caractère limitatif. Les quantités y sont données en % en poids sauf mention contraire.

### EXEMPLES DE PREPARATION:

Préparation des esters (méth)acryliques éthoxylés.

Ils peuvent être notamment obtenus par action de (méth)acrylate de glycidyle, ou d'acide (méth)acrylique, ou d'un (méth)acrylate d'alkyle, ou d'un halogénure de (méth)acryloyle sur un alcool gras éthoxylé. On peut citer, à titre d'exemples non limitatifs, les préparations suivantes :
a) à partir du méthacrylate de glycidyle et du GENAPOL T-250
b) à partir de l'acide (méth)acrylique et du GENAPOL UD-070
c) à partir du (méth)acrylate de méthyle et du GENAPOL LA-090
d) à partir du chlorure de (méth)acryloyle et du GENAPOL UD-070.

a) Dans un réacteur tricol d'un litre équipé d'un agitateur, d'un thermomètre et d'un condensateur à reflux, on introduit 500g de Genapol T-250 et 75g de méthacrylate de glycidyle. On chauffe le mélange réactionnel à la température de 100°C pendant 2 heures, et on élimine l'excès de méthacrylate de glycidyle par distillation sous pression réduite. Le monomère obtenu peut être utilisé pour la polymérisation sans purification ultérieure.
b) Dans un réacteur tricol d'un litre équipé d'un agitateur, d'un thermomètre et d'un condensateur à reflux, on introduit 500g de Genapol UD-070, 100g d'acide (méth)acrylique et de l'acide p-toluènesulfonique comme catalyseur. On chauffe le mélange réactionnel au reflux pendant 2 heures, et l'on sépare l'excès d'acide et d'eau formée au cours de la réaction par distillation sous pression réduite. Le monomère obtenu peut être utilisé pour la polymérisation sans purification ultérieure.
c) Dans un réacteur tricol d'un litre équipé d'un agitateur, d'un thermomètre et d'un condensateur à reflux, on introduit 500g de Genapol LA-090, 100g de (méth)acrylate de méthyle et 20g de tétraisopropoxyde de titane. On chauffe le mélange réactionnel au reflux pendant 2 heures, et après séparation par distillation de l'alcool formé, on distille l'ester qui reste sous pression réduite.
   Le monomère obtenu peut être utilisé pour la polymérisation sans purification ultérieure.
d) Dans un réacteur tricol d'un litre équipé d'un agitateur, d'un thermomètre et d'un condensateur à reflux, on introduit 500g de Genapol UD-070, 110g de chlorure de (méth)acryloyle et 50g de carbonate de sodium. On chauffe le mélange réactionnel au reflux pendant 2 heures, et l'on sépare l'excès de chlorure d'acide par distillation sous pression réduite. Le monomère obtenu peut être utilisé pour la polymérisation sans purification ultérieure.

### Polymérisation selon la méthode de précipitation dans le tert-butanol

Dans un réacteur de 2 litres muni d'un condensateur à reflux, d'une prise de gaz, d'un thermomètre et d'un agitateur, on introduit 500ml de tert-butanol, et la quantité calculée d'AMPS. On neutralise le mélange en introduisant du NH3, et on ajoute le monomère préparé ci-dessus au mélange réactionnel. On rend le mélange réactionnel inerte par passage d'azote ou d'argon et lorsque la température intérieure a atteint 60°C, on introduit l'initiateur (AIBN) pour amorcer la polymérisation.
Après quelques minutes, le polymère ainsi préparé précipite. On porte le mélange à reflux pendant 2 heures, et on sépare le polymère du solvant par filtration à la trompe, puis on le sèche sous pression réduite.
De la façon décrite ci-dessus, on a préparé les polymères suivants : (à partir des réactants suivants en des quantités exprimées en grammes)

| | | | | |
|---|---|---|---|---|
| Méthacrylate de Genapol T-250 | 10 | 20 | 30 | 97 |
| AMPS neutralisé par NH3..... | 90 | 80 | 90 | 3 |
| Méthylène-bis-acrylamide (réticulant).. | | | 1,5 | |
| Méthacrylate d'allyle (réticulant)......... | | 1,7 | | |
| TMPTA (réticulant)..... | 1,8 | | | 1,8 |
| Azobisisobutyronitrile (initiateur)...... | | | 1 | |
| Peroxyde dilauryle (initiateur)..... | 1 | 1 | | 1 |
| Tert-butanol..... | 300 | 300 | 300 | 300 |

### Exemple de formulation :

### EXEMPLE 1

On a préparé une composition d'après-shampooing à rincer :
- Copolymère réticulé par du méthylène-bis-acrylamide constitué de 75% en poids de motifs AMPS neutralisés par NH₃ et de 25% en poids de motifs de formule (III)
   dans laquelle R₁=H, R₄=C₁₆-C₁₈ et x=25 0,75 g
- Amodiméthicone en émulsion aqueuse
   (DC939 de DOW CORNING) 1 gMA
- Eau déminéralisée qsp 100 g

La -composition se répartit facilement sur la chevelure. Elle se rince bien et les cheveux sont doux et se démêlent bien.

**Exemples comparatifs** : on a reproduit l'exemple 1 en remplaçant le polymère amphiphile utilisé selon l'invention, par des polymères de l'art antérieur : Hostacerin AMPS et Sepigel 305 (Mélange Polyacrylamide/C13-14 Isoparaffin/Laureth-7).

Les compositions de l'art antérieur se répartissent moins bien sur l'ensemble de la chevelure, elles se rincent moins facilement et le dépôt de la silicone sur les cheveux est moins important.

### EXEMPLE 2

Des résultats similaires ont été obtenus en remplaçant le copolymère acide acrylamido-2-méthyl-2-propane-sulfonique/n-dodécylacrylamide neutralisé par la soude de l'exemple ci-dessus par un copolymère réticulé par du méthacrylate d'allyle constitué de 90% en poids de motifs AMPS neutralisés par NH₃ et de 10% en poids de motifs méthacrylate de GENAPOL T-250 [motifs de formule (III) dans laquelle R₁=CH₃, R₄=C₁₆-C₁₈ et x=25], ou par un copolymère réticulé par du méthacrylate d'allyle constitué de 80% en poids de motifs AMPS neutralisés par NH₃ et de 20% en poids de motifs méthacrylate de GENAPOL T-250 [motifs de formule (III) dans laquelle R₁=CH₃, R₄=C₁₆-C₁₈ et x=25].

## Revendications

1. Composition pour application topique, comportant une phase aqueuse comprenant au moins une silicone choisie parmi les résines de silicones non renforcées, les polyorganosiloxanes modifiés par des groupements organofonctionnels non polyoxyalkylénés ainsi que leurs mélanges,
et au moins un polymère amphiphile comportant au moins un monomère à insaturation éthylénique à groupement sulfonique, sous forme libre ou partiellement ou totalement neutralisée et comprenant au moins une partie hydrophobe choisi parmi les copolymères amphiphiles de 2-acrylamido-2-méthylpropane-sulfonique (AMPS) sous forme libre ou partiellement ou totalement neutralisée et d'au moins un monomère hydrophobe à insaturation éthylénique comportant au moins une partie hydrophobe choisi parmi les acrylates ou les acrylamides de formule (I) suivante : 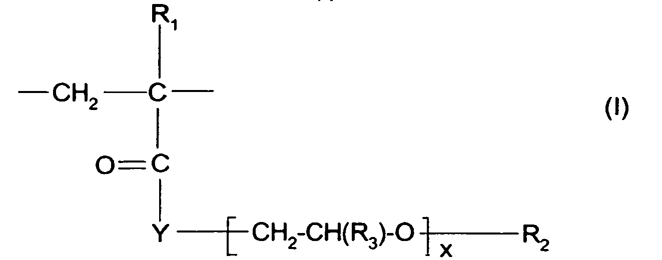 dans laquelle R₁ et R₃, identiques ou différents, désignent un atome d'hydrogène ou un radical alkyle linéaire ou ramifié en C₁-C₆ (de préférence méthyle); Y désigne O ou NH ; R₂ désigne un radical hydrocarboné hydrophobe comportant au moins de 6 à 50 atomes de carbone et plus préférentiellement de 6 à 22 atomes de carbone et encore plus préférentiellement de 6 à 18 atomes de carbone et plus particulièrement de 12 à 18 atomes de carbone ; x désigne un nombre de moles d'oxyde d'alkylène et varie de 3 à 100.

2. Composition selon l'une quelconque des revendications 1, **caractérisée par le fait que** les polymères amphiphiles sont neutralisés partiellement ou totalement par une base minérale ou organique.

3. Composition selon l'une quelconque des revendications 1 ou 2, **caractérisée par le fait que** les polymères amphiphiles ont un poids moléculaire moyen en nombre allant de 1000 à 20 000 000 g/mole.

4. Composition selon la revendication 3, **caractérisée par le fait que** le poids moléculaire moyen en nombre varie de 20 000 à 5 000 000 g/mote.

5. Composition selon la revendication 4, **caractérisée par le fait que** le poids moléculaire moyen en nombre varie de 100 000 à 1 500 000 g/mole.

6. Composition selon l'une quelconque des revendications précédentes, **caractérisée par le fait qu'**une solution aqueuse à 1% en poids desdits polymères présente à la température de 25°C une viscosité mesurée au viscosimètre Brookfield, aiguille 7, allant de 20 000 mPa.s à 100 000 mPa.s.

7. Composition selon l'une quelconque des revendications précédentes, **caractérisée par le fait que** les polymères amphiphiles sont préparés par polymérisation radicalaire par précipitation dans le tert-butanol.

8. Composition selon l'une quelconque des revendications précédentes, **caractérisée par le fait que** les polymères amphiphiles sont réticulés ou non-réticulés.

9. Composition selon la revendication 8, **caractérisée par le fait que** les polymères amphiphiles sont réticulés.

10. Composition selon la revendication 9, **caractérisée par le fait que** le ou les agents de réticulation sont choisis parmi les composés à polyinsaturation oléfinique.

11. Composition selon la revendication 10, **caractérisée par le fait que** le ou les agents de réticulation sont choisis parmi le méthylène-bis-acrylamide, le méthacrylate d'allyle ou le triméthylol propane triacrylate (TMPTA).

12. Composition selon l'une quelconque des revendications 9 à 11 à 15, **caractérisée par le fait que** le taux de réticulation varie de préférence de 0,01 à 10% en moles et plus particulièrement de 0,2 à 2% en moles par rapport au polymère.

13. Composition selon l'une quelconque des revendications 1 à 12, **caractérisée par le fait que** les polymères amphiphiles d'AMPS contiennent en plus au moins un monomère à insaturation éthylénique ne comportant pas de chaîne grasse.

14. Composition selon la revendication 13, **caractérisée par le fait que** le monomère à insaturation éthylénique ne comportant pas de chaîne grasse est choisi parmi les acides (méth)acryliques et leurs dérivés alkyl substitués en β, et leurs esters obtenus avec des monoalcools ou des mono- ou polyalkylèneglycols, ou bien parmi les (méth)acrylamides, la vinylpyrrolidone, l'anhydride maléique, l'acide itaconique ou l'acide maléique, ou les mélanges de ces composés.

15. Composition selon la revendication 1, **caractérisée par le fait que** le radical hydrophobe R₂ est choisi parmi les radicaux alkyles en C₆-C₁₈, linéaires, ramifiés ou cycliques ; les radicaux alkylperfluorés en C₆-C₁₈ ; le radical cholestéryle ou un ester de cholestérol ; tes groupes polycycliques aromatiques.

16. Composition selon la revendication 1, **caractérisée par le fait que** la chaîne polyoxyalkylénée est constituée de motifs oxyde d'éthylène et/ou de motifs oxyde de propylène.

17. Composition selon la revendication 16, **caractérisée par le fait que** la chaîne polyoxyalkylénée est constituée uniquement de motifs oxyde d'éthylène.

18. Composition selon l'une quelconque des revendications 1 à 17, **caractérisée par le fait que** le nombre de motifs oxyalkylénés varie de 3 à 50.

19. Composition selon la revendication 18, **caractérisée par le fait que** le nombre de motifs oxyalkylénés varie de 7 à 25.

20. Composition selon les revendications 1 à 19, **caractérisée par le fait que** le polymère amphiphile d'AMPS est choisi parmi les copolymères constitués de motifs acide 2-acrylamido-2-méthylpropane-sulfonique (AMPS) de formule (II) suivante : 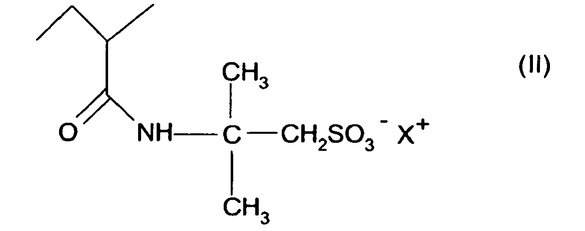 dans laquelle X⁺ est un proton, un cation de métal alcalin, un cation alcalino-terreux ou l'ion ammonium,
et de motifs de formule (III) suivante : 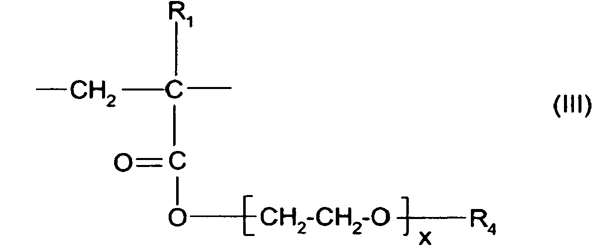 dans laquelle x désigne un nombre entier variant de 3 à 100, de préférence de 5 à 80 et plus préférentiellement de 7 à 25 ; R₁ a la même signification que celle indiquée ci-dessus dans la formule (I) et R₄ désigne un alkyle linéaire ou ramifié en C₆-C₂₂ et plus préférentiellement en C₁₀-C₂₂.

21. Composition selon la revendication 20, **caractérisée par le fait que** x = 25 , R₁ est méthyle et R₄ est n-dodécyle.

22. Composition selon la revendication 20 ou 21, **caractérisée par le fait que** la proportion molaire en % des motifs de formule (I) ou des motifs de formule (III) dans les polymères varie de 50,1 à 99,9%.

23. Composition selon la revendication 20 ou 21 **caractérisée par le fait que** la proportion molaire en % des motifs de formule (I) ou des motifs de formule (III) dans les polymères varie de 0,1 à 50%.

24. Composition selon l'une quelconque des revendications précédentes, **caractérisée par le fait que** les polymères amphiphiles sont présents dans des concentrations allant de 0,01 à 30% en poids, plus préférentiellement de 0,1 à 10% en poids, encore plus préférentiellement de 0,1 à 5% en poids, et plus particulièrement encore de 0,5 à 2% en poids par rapport au poids total de la composition.

25. Composition selon l'une quelconque des revendications précédentes, **caractérisée par le fait que** les résines sont choisies parmi les résines constituées d'unités : R₃ Si O_{1/2}, R₂ Si O_{2/2}, R Si O_{3/2}, Si O_{4/2}
dans lesquelles R représente un groupement hydrocarboné ayant de 1 à 16 atomes de carbone ou un groupement phényle ;
les silicones organo modifiées sont choisies parmi les silicones comportant dans leur structure un ou plusieurs groupements organofonctionnels fixés par l'intermédiaire d'un radical hydrocarboné.

26. Composition selon l'une quelconque des revendications précédentes, **caractérisée par le fait que** les silicones organomodifiées sont choisies parmi les polyorganosiloxanes comportant :
a) des groupements aminés substitués ou non ;
b) des groupements thiols ;
c) des groupements alcoxylés pendants,
d) des groupements hydroxyalkyle pendants,
e) des groupements acyloxyalkyle,
f) des groupements alkyl carboxyliques,
g) des groupements 2-hydroxyalkylsulfonates,
h) des groupements 2-hydroxyalkylthiosulfonates,
i) des groupements hydroxyacylamino,
j) des groupements ammonium quaternaires.

27. Composition selon l'une quelconque des revendications 1 à 26, **caractérisée par le fait que** les silicones sont choisies parmi les résines d'organopolysiloxanes, les polysiloxanes à groupements aminés.

28. Composition selon l'une quelconque des revendications précédentes, **caractérisée en ce que** la silicone est présente à une concentration comprise entre 0,001 % et 20 % en poids par rapport au poids total de la composition.

29. Composition selon l'une quelconque des revendications précédentes, **caractérisée en ce que** le milieu physiologiquement acceptable est constitué d'eau ou d'eau et d'au moins un solvant organique choisi dans le groupe constitué par les solvants organiques hydrophiles, les solvants organiques lipophiles, les solvants amphiphiles ou leurs mélanges.

30. Composition selon l'une quelconque des revendications précédentes, **caractérisée en ce qu'**elle constitue une crème de soin du visage, un lait corporel, un gel de douche, un gel de bain, une composition de coloration ou de décoloration des cheveux, une composition de déformation permanente des cheveux, une composition de nettoyage et/ou de démaquillage du visage, un shampooing, un après-shampooing.

31. Utilisation cosmétique de la composition selon l'une quelconque des revendications 1 à 30, pour le traitement et le soin de la peau du visage et/ou du corps, des muqueuses, du cuir chevelu et/ou des cheveux.

32. Utilisation de la composition selon l'une quelconque des revendications 1 à 30, pour la fabrication d'une composition destinée à traiter la peau sensible et/ou le cuir chevelu sensible.

33. Procédé de traitement cosmétique de la peau, du cuir chevelu, des cheveux, des ongles et/ou des muqueuses, **caractérisé par le fait que** l'on applique sur la peau, le cuir chevelu, les cheveux, les ongles et/ou les muqueuses, une composition selon l'une quelconque des revendications 1 à 30.

34. Utilisation d'un polymère amphiphile comportant au moins un monomère à insaturation éthylènique à groupement sulfonique, sous forme libre ou partiellement ou totalement neutralisée et comprenant au moins une partie hydrophobe tel que défini à l'une quelconque des revendications 1 à 23, pour stabiliser une composition cosmétique ou dermatologique contenant au moins une silicone et/ou augmenter son dépôt.

## Claims

1. Composition for topical application, containing an aqueous phase comprising at least one silicone chosen from non-reinforced silicone resins and polyorganosiloxanes modified with non-polyoxyalkylenated organofunctional groups, and mixtures thereof,
and at least one amphiphilic polymer containing at least one ethylenically unsaturated monomer containing a sulfonic group, in free or partially or totally neutralized form and comprising at least one hydrophobic portion chosen from amphiphilic copolymers of 2-acrylamido-2-methylpropanesulfonic (AMPS) in free or partially or totally neutralized form and of at least one ethylenically unsaturated hydrophobic monomer containing at least one hydrophobic portion chosen from the acrylates and acrylamides of formula (I) below: 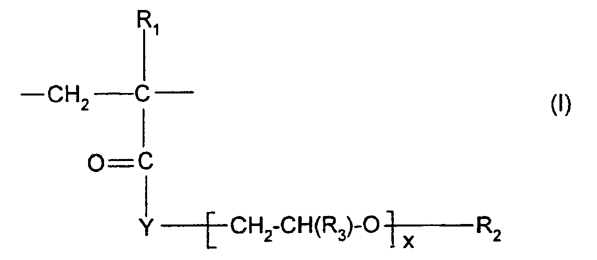 in which R₁ and R₃, which may be identical or different, denote a hydrogen atom or a linear or branched C₁-C₆ alkyl radical (preferably methyl); Y denotes O or NH; R₂ denotes a hydrophobic hydrocarbon-based radical containing at least from 6 to 50 carbon atoms, more preferentially from 6 to 22 carbon atoms, even more preferentially from 6 to 18 carbon atoms and more particularly from 12 to 18 carbon atoms; x denotes a number of moles of alkylene oxide and ranges from 3 to 100.

2. Composition according to Claim 1, **characterized in that** the amphiphilic polymers are partially or totally neutralized with a mineral or organic base.

3. Composition according to either of Claims 1 and 2, **characterized in that** the amphiphilic polymers have a number-average molecular weight ranging from 1000 to 20 000 000 g/mol.

4. Composition according to Claim 3, **characterized in that** the number-average molecular weight ranges from 20 000 to 5 000 000 g/mol.

5. Composition according to Claim 4, **characterized in that** the number-average molecular weight ranges from 100 000 to 1 500 000 g/mol.

6. Composition according to any one of the preceding claims, **characterized in that** an aqueous solution containing 1% by weight of the said polymers has, at a temperature of 25°C, a viscosity, measured using a Brookfield viscometer with a No. 7 needle, ranging from 20 000 mPa.s to 100 000 mPa.s.

7. Composition according to any one of the preceding claims, **characterized in that** the amphiphilic polymers are prepared by free-radical precipitation polymerization in tert-butanol.

8. Composition according to any one of the preceding claims, **characterized in that** the amphiphilic polymers are crosslinked or non-crosslinked.

9. Composition according to Claim 8, **characterized in that** the amphiphilic polymers are crosslinked.

10. Composition according to Claim 9, **characterized in that** the crosslinking agent(s) is (are) chosen from polyolefinically unsaturated compounds.

11. Composition according to Claim 10, **characterized in that** the crosslinking agent(s) is (are) chosen from methylenebisacrylamide, allyl methacrylate and trimethylolpropane triacrylate (TMPTA).

12. Composition according to any one of Claims 9 to 11, **characterized in that** the degree of crosslinking preferably ranges from 0.01 mol% to 10 mol% and more particularly from 0.2 mol% to 2 mol% relative to the polymer.

13. Composition according to any one of Claims 1 to 12, **characterized in that** the amphiphilic polymers of AMPS also contain at least one ethylenically unsaturated monomer not comprising a fatty chain.

14. Composition according to Claim 13, **characterized in that** the ethylenically unsaturated monomer not comprising a fatty chain is chosen from (meth)acrylic acids and β-substituted alkyl derivatives thereof, and esters thereof obtained with monoalcohols or mono- or polyalkylene glycols, or alternatively from (meth)-acrylamides, vinylpyrrolidone, maleic anhydride, itaconic acid or maleic acid, or mixtures of these compounds.

15. Composition according to Claim 1, **characterized in that** the hydrophobic radical R₂ is chosen from linear, branched or cyclic C₆-C₁₈ alkyl radicals; C₆-C₁₈ alkylperfluoro radicals; the cholesteryl radical or a cholesterol ester; aromatic polycyclic groups.

16. Composition according to Claim 1, **characterized in that** the polyoxyalkylene chain consists of ethylene oxide units and/or propylene oxide units.

17. Composition according to Claim 16, **characterized in that** the polyoxyalkylene chain consists solely of ethylene oxide units.

18. Composition according to any one of Claims 1 to 17, **characterized in that** the number of oxyalkylene units ranges from 3 to 50.

19. Composition according to Claim 18, **characterized in that** the number of oxyalkylene units ranges from 7 to 25.

20. Composition according to Claims 1 to 19, **characterized in that** the amphiphilic polymer of AMPS is chosen from copolymers consisting of 2-acrylamido-2-methylpropanesulfonic acid (AMPS) units of formula (II) below: 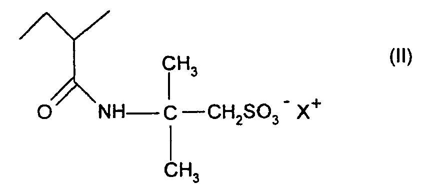 in which X⁺ is a proton, an alkali metal cation, an alkaline-earth metal cation or the ammonium ion,
and of units of formula (III) below: 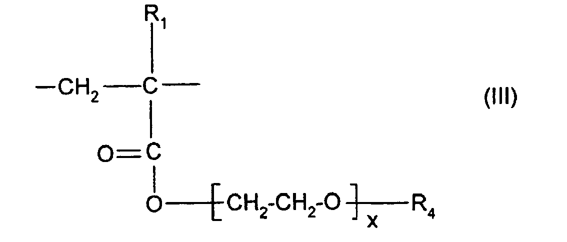 in which x denotes an integer ranging from 3 to 100, preferably from 5 to 80 and more preferentially from 7 to 25; R₁ has the same meaning as that given above in formula (I) and R₄ denotes a linear or branched C₆-C₂₂ and more preferentially C₁₀-C₂₂ alkyl.

21. Composition according to Claim 20, **characterized in that** x = 25, R₁ is methyl and R₄ is n-dodecyl.

22. Composition according to Claim 20 or 21, **characterized in that** the percentage molar proportion of units of formula (I) or of units of formula (III) in the polymers ranges from 50.1% to 99.9%.

23. Composition according to Claim 20 or 21, **characterized in that** the percentage molar proportion of units of formula (I) or of units of formula (III) in the polymers ranges from 0.1% to 50%.

24. Composition according to any one of the preceding claims, **characterized in that** the amphiphilic polymers are present in concentrations ranging from 0.01% to 30% by weight, more preferentially from 0.1% to 10% by weight, even more preferentially from 0.1% to 5% by weight and even more particularly from 0.5% to 2% by weight relative to the total weight of the composition.

25. Composition according to any one of the preceding claims, **characterized in that** the resins are chosen from resins consisting of the following units: R₃SiO_{1/2}, R₂SiO_{2/2}, RSiO_{3/2}, SiO_{4/2}
in which R represents a hydrocarbon-based group containing from 1 to 16 carbon atoms or a phenyl group; the organomodified silicones are chosen from silicones comprising in their structure one or more organofunctional groups attached via a hydrocarbon-based radical.

26. Composition according to any one of the preceding claims, **characterized in that** the organomodified silicones are chosen from polyorganosiloxanes comprising:
a) substituted or unsubstituted amine groups;
b) thiol groups;
c) pendent alkoxy groups;
d) pendent hydroxyalkyl groups;
e) acyloxyalkyl groups;
f) alkylcarboxylic groups;
g) 2-hydroxyalkylsulfonate groups;
h) 2-hydroxyalkylthiosulfonate groups;
i) hydroxyacylamino groups;
j) quaternary ammonium groups.

27. Composition according to any one of Claims 1 to 26, **characterized in that** the silicones are chosen from organopolysiloxane resins and polysiloxanes containing amine groups.

28. Composition according to any one of the preceding claims, **characterized in that** the silicone is present in a concentration of between 0.001% and 20% by weight relative to the total weight of the composition.

29. Composition according to any one of the preceding claims, **characterized in that** the physiologically acceptable medium consists of water or of water and at least one organic solvent chosen from the group consisting of hydrophilic organic solvents, lipophilic organic solvents and amphiphilic solvents, or mixtures thereof.

30. Composition according to any one of the preceding claims, **characterized in that** it is a facial care cream, a body milk, a shower gel, a bath gel, a hair colouring or bleaching composition, a hair permanent-reshaping composition, a facial cleansing and/or makeup-removing composition, a shampoo or a hair conditioner.

31. Cosmetic use of the composition according to any one of Claims 1 to 30, for treating and caring for facial and/or bodily skin, mucous membranes, the scalp and/or the hair.

32. Use of the composition according to any one of Claims 1 to 30, for the manufacture of a composition for treating sensitive skin and/or a sensitive scalp.

33. Cosmetic process for treating the skin, the scalp, the hair, the nails and/or mucous membranes, **characterized in that** a composition according to any one of Claims 1 to 30 is applied to the skin, the scalp, the hair, the nails and/or mucous membranes.

34. Use of an amphiphilic polymer comprising at least one ethylenically unsaturated monomer containing a sulfonic group, in free or partially or totally neutralized form and comprising at least one hydrophobic portion as defined in any one of Claims 1 to 23, for stabilizing a cosmetic or dermatological composition containing at least one silicone and/or for increasing its deposition.

## Patentansprüche

1. Zusammensetzung für die topische Anwendung, die eine wässrige Phase umfasst, die mindestens ein Silicon, das unter den nicht verstärkten Siliconharzen, den Polyorganosiloxanen, die mit nicht polyalkoxylierten organofunktionellen Gruppen modifiziert sind, sowie deren Gemischen ausgewählt ist,
und mindestens ein amphiphiles Polymer enthält, das mindestens ein ethylenisch ungesättigtes Monomer mit Sulfonsäuregruppe in freier oder teilweise oder vollständig neutralisierter Form enthält und das mindestens einen hydrophoben Teil umfasst, das ausgewählt wird unter den amphiphilen Copolymeren aus 2-Acrylamido-2-methylpropansulfonsäure (AMPS) in freier oder teilweise oder vollständig neutralisierter Form und mindestens einem ethylenisch ungesättigten hydrophoben Monomer, das mindestens einen hydrophoben Teil aufweist, das unter den Acrylaten oder den Acrylamiden der folgenden Formel (I) 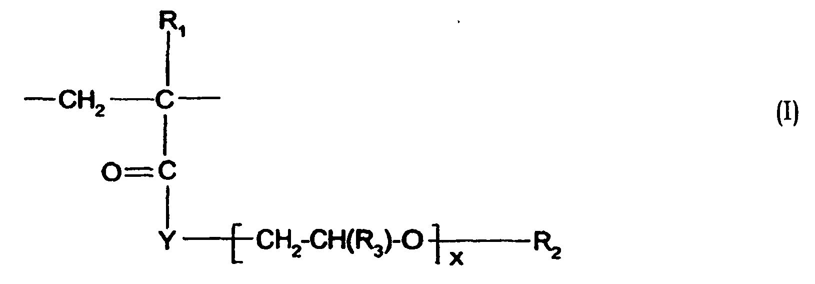 ausgewählt wird, worin bedeuten: R₁ und R₃, gleich oder verschieden, ein Wasserstoffatom oder einen geradkettigen oder verzweigten C₁₋₆-Alkylrest (vorzugsweise Methyl); Y Sauerstoff oder NH; R₂ einen hydrophoben Kohlenwasserstoffrest, der mindestens 6 bis 50 Kohlenstoffatome und bevorzugter 6 bis 22 Kohlenstoffatome und noch bevorzugter 6 bis 18 Kohlenstoffatome und vor allem 12 bis 18 Kohlenstoffatome aufweist; x die Alkylenoxid-Molzahl, die im Bereich von 3 bis 100 variiert.

2. Zusammensetzung nach Anspruch 1, **dadurch gekennzeichnet, dass** die amphiphilen Polymere teilweise oder vollständig mit einer anorganischen oder organischen Base neutralisiert sind.

3. Zusammensetzung nach Anspruch 1 oder 2, **dadurch gekennzeichnet, dass** die amphiphilen Polymere ein Zahlenmittel des Molekulargewichts aufweisen, das im Bereich von 1000 bis 20 000 000 g/mol liegt.

4. Zusammensetzung nach Anspruch 3, **dadurch gekennzeichnet, dass** das Zahlenmittel des Molekulargewichts im Bereich von 20 000 bis 5 000 000 g/mol liegt.

5. Zusammensetzung nach Anspruch 4, **dadurch gekennzeichnet, dass** das Zahlenmittel des Molekulargewichts im Bereich von 100 000 bis 1 500 000 g/mol liegt.

6. Zusammensetzung nach einem der vorhergehenden Ansprüche, **dadurch gekennzeichnet, dass** eine 1%ige wässrige Lösung (Gew.-%) der erwähnten Polymere bei einer Temperatur von 25 °C eine Viskosität aufweist, die mit einem Brookfield-Viskosimeter, Nadel 7 gemessen wird, die im Bereich von 20 000 bis 100 000 mPa·s liegt.

7. Zusammensetzung nach einem der vorhergehenden Ansprüche, **dadurch gekennzeichnet, dass** die amphiphilen Polymere durch radikalische Polymerisation durch Fällung in tert.-Butanol hergestellt werden.

8. Zusammensetzung nach einem der vorhergehenden Ansprüche, **dadurch gekennzeichnet, dass** die amphiphilen Polymere vernetzt oder nicht vernetzt sind.

9. Zusammensetzung nach Anspruch 8, **dadurch gekennzeichnet, dass** die amphiphilen Polymere vernetzt sind.

10. Zusammensetzung nach Anspruch 9, **dadurch gekennzeichnet, dass** das oder die Vernetzungsmittel unter den mehrfach olefinisch ungesättigten Verbindungen ausgewählt werden.

11. Zusammensetzung nach Anspruch 10, **dadurch gekennzeichnet, dass** das oder die Vernetzungsmittel unter Methylen-bis-acrylamid, Allylmethacrylat und Trimethylolpropantriacrylat (TMPTA) ausgewählt werden.

12. Zusammensetzung nach einem der Ansprüche 9 bis 11, **dadurch gekennzeichnet, dass** der Vernetzungsgrad vorzugsweise im Bereich von 0,01 bis 10 Mol-% und vor allem im Bereich von 0,2 bis 2 Mol-%, bezogen auf das Polymer, liegt.

13. Zusammensetzung nach einem der Ansprüche 1 bis 12, **dadurch gekennzeichnet, dass** die amphiphilen Polymere von AMPS außerdem mindestens ein ethylenisch ungesättigtes Monomer enthalten, das keine Fettkette aufweist.

14. Zusammensetzung nach Anspruch 13, **dadurch gekennzeichnet, dass** das ethylenisch ungesättigte Monomer, das keine Fettkette aufweist, unter (Meth)acrylsäure und deren in β-Position alkylsubstituierten Derivaten und deren Estern, die mit einwertigen Alkoholen oder Mono- oder Polyalkylenglykolen erhalten werden, und (Meth)acrylamid, Vinylpyrrolidon, Maleinsäureanhydrid, Itaconsäure oder Maleinsäure und den Gemischen dieser Verbindungen ausgewählt wird.

15. Zusammensetzung nach Anspruch 1, **dadurch gekennzeichnet, dass** der hydrophobe Rest R₂ unter den geradkettigen, verzweigten oder cyclischen C₆₋₁₈-Alkylresten, den perfluorierten C₆₋₁₈-Alkylresten, dem Cholesterylrest und den Cholesterolestern, den polycyclischen aromatischen Gruppen ausgewählt wird.

16. Zusammensetzung nach Anspruch 1, **dadurch gekennzeichnet, dass** die polyalkoxylierte Kette aus Ethylenoxid- und/oder Propylenoxideinheiten besteht.

17. Zusammensetzung nach Anspruch 16, **dadurch gekennzeichnet, dass** die polyalkoxylierte Kette ausschließlich aus Ethylenoxideinheiten besteht.

18. Zusammensetzung nach einem der Ansprüche 1 bis 17, **dadurch gekennzeichnet, dass** die Zahl der Alkylenoxideinheiten im Bereich von 3 bis 50 liegt.

19. Zusammensetzung nach einem der Ansprüche 1 bis 18, **dadurch gekennzeichnet, dass** die Zahl der Alkylenoxideinheiten im Bereich von 7 bis 25 liegt.

20. Zusammensetzung nach einem der Ansprüche 1 bis 19, **dadurch gekennzeichnet, dass** das amphiphile Polymer von AMPS unter den Copolymeren ausgewählt wird, die aus 2-Acrylamido-2-methylpropansulfonsäure-Einheiten (AMPS-Einheiten) der folgenden Formel (II) 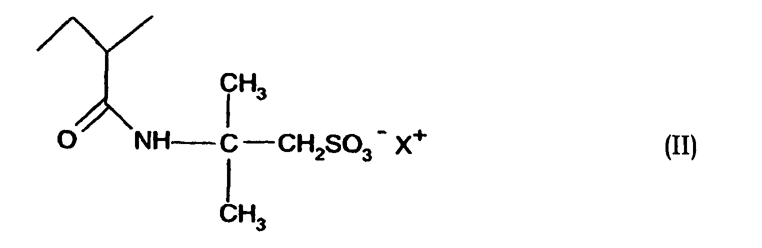 worin X⁺ ein Proton, ein Alkalimetallkation, ein Erdalkalimetallkation oder ein Ammoniumion bedeutet,
und aus Einheiten der folgenden Formel (III) 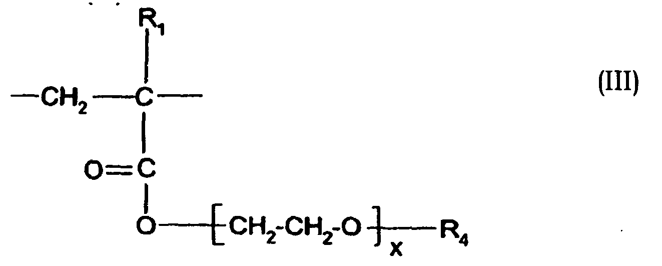 bestehen, worin x eine ganze Zahl bedeutet, die im Bereich von 3 bis 100, vorzugsweise 5 bis 80 und noch bevorzugter 7 bis 25 liegt; worin R₁ die Bedeutung hat, die weiter oben in der Formel (I) angegeben wird, und worin R₄ einen geradkettigen oder verzweigten C₆₋₂₂-Alkylrest und noch bevorzugter C₁₀₋₂₂-Alkylrest bedeutet.

21. Zusammensetzung nach Anspruch 20, **dadurch gekennzeichnet, dass** x = 25 ist, R₁ Methyl und R₄ n-Dodecyl bedeutet.

22. Zusammensetzung nach Anspruch 20 oder 21, **dadurch gekennzeichnet, dass** der in Mol-% angegebene Anteil der Einheiten der Formel (I) oder der Einheiten der Formel (III) in den Polymeren im Bereich von 50,1 bis 99,9 % liegt.

23. Zusammensetzung nach Anspruch 20 oder 21, **dadurch gekennzeichnet, dass** der in Mol-% angegebene Anteil der Einheiten der Formel (I) oder der Einheiten der Formel (III) in den Polymeren im Bereich von 0, 1 bis 50 % liegt.

24. Zusammensetzung nach einem der vorhergehenden Ansprüche, **dadurch gekennzeichnet, dass** die amphiphilen Polymere in Konzentrationen enthalten sind, die im Bereich von 0,01 bis 30 Gew.-%, bevorzugter 0,1 bis 10 Gew.-%, noch bevorzugter 0,1 bis 5 Gew.-% und vor allem im Bereich von 0,5 bis 2 Gew.-%, bezogen auf das Gesamtgewicht der Zusammensetzung, liegen.

25. Zusammensetzung nach einem der vorhergehenden Ansprüche, **dadurch gekennzeichnet, dass** die Harze unter den Harzen ausgewählt werden, die aus Einheiten: R₃SiO_{1/2}, R₂SiO_{2/2}, RSiO_{3/2}, SiO_{4/2} bestehen, worin R einen Kohlenwasserstoffrest, der 1 bis 16 Kohlenstoffatome aufweist, oder eine Phenylgruppe bedeutet; und dass die organisch modifizierten Silicone unter den Siliconen ausgewählt werden, die in ihrer Struktur eine oder mehrer organofunktionelle Gruppen aufweisen, die über einen Kohlenwasserstoffrest angebunden sind.

26. Zusammensetzung nach einem der vorhergehenden Ansprüche, **dadurch gekennzeichnet, dass** die organisch modifizierten Silicone unter den Polyorganosiloxanen ausgewählt werden, die aufweisen:
a) substituierte oder nicht substituierte Aminogruppen;
b) Thiolgruppen;
c) Alkoxyseitengruppen;
d) Hydroxyalkylseitengruppen,
e) Acyloxyalkylgruppen,
f) Alkylcarboxygruppen,
g) 2-Hydroxyalkylsulfonat-Gruppen,
h) 2-Hydroxyalkylthiosulfonat-Gruppen,
i) Hydroxyacylamino-Gruppen,
j) quartäre Ammoniumgruppen.

27. Zusammensetzung nach einem der Ansprüche 1 bis 26, **dadurch gekennzeichnet, dass** die Silicone unter den Polyorganosiloxanharzen, den Polysiloxanen mit Aminogruppen ausgewählt werden.

28. Zusammensetzung nach einem der vorhergehenden Ansprüche, **dadurch gekennzeichnet, dass** das Silicon in einer Konzentration enthalten ist, die im Bereich von 0,001 bis 20 Gew.-%, bezogen auf das Gesamtgewicht der Zusammensetzung, liegt.

29. Zusammensetzung nach einem der vorhergehenden Ansprüche, **dadurch gekennzeichnet, dass** das physiologisch akzeptable Medium aus Wasser oder Wasser und mindestens einem organischen Lösemittel besteht, das unter den hydrophilen organischen Lösemitteln, den lipophilen organischen Lösemitteln, den amphiphilen Lösemitteln und deren Gemischen ausgewählt wird.

30. Zusammensetzung nach einem der vorhergehenden Ansprüche, **dadurch gekennzeichnet, dass** sie eine Creme für die Gesichtspflege, eine Körpermilch, ein Duschgel, ein Badegel, eine Zusammensetzung zur Färbung oder Entfärbung der Haare, eine Zusammensetzung für die Dauerwellverformung der Haare, eine Zusammensetzung für die Reinigung und/oder zum Abschminken des Gesichts, ein Haarwaschmittel, einen Konditioner bildet.

31. Kosmetische Verwendung der Zusammensetzung nach einem der Ansprüche 1 bis 30 für die Behandlung und die Pflege der Haut des Gesichts und/oder des Körpers, der Schleimhäute, der Kopfhaut und/oder der Haare.

32. Verwendung der Zusammensetzung nach einem der Ansprüche 1 bis 30 für die Herstellung einer Zusammensetzung, die für die Behandlung von empfindlicher Haut und/oder empfindlicher Kopfhaut vorgesehen ist.

33. Verfahren zur kosmetischen Behandlung der Haut, der Kopfhaut, der Haare, der Nägel und/oder der Schleimhäute, **dadurch gekennzeichnet, dass** eine Zusammensetzung nach einem der Ansprüche 1 bis 30 auf die Haut, die Kopfhaut, die Haare, die Nägel und/oder die Schleimhäute aufgetragen wird.

34. Verwendung eines amphiphilen Polymers, das mindestens ein ethylenisch ungesättigtes Monomer mit Sulfonsäuregruppe in freier oder teilweise oder vollständig neutralisierter Form enthält und das mindestens einen hydrophoben Teil aufweist, das wie in einem der Ansprüche 1 bis 23 definiert ist, für die Stabilisierung einer kosmetischen oder dermatologischen Zusammensetzung, die mindestens ein Silicon enthält, und/oder für die Verbesserung Abscheidung des Silicons.
